# EUROPÄISCHE PATENTSCHRIFT

(11) **EP 3 039 593 B1**
(45) Veröffentlichungstag und Bekanntmachung des Hinweises auf die Patenterteilung: **16.06.2021**
(21) Anmeldenummer: 14792371.8
(22) Anmeldetag: 29.08.2014
(51) Int. Cl.: A61M 16/00, G16H 40/63

(54) **BEDIEN- UND INFORMATIONSSYSTEM FÜR EIN BEATMUNGSGERÄT**
OPERATING- AND INFORMATION SYSTEM FOR A BREATHING APPARATUS
SYSTÈME DE COMMANDE ET D'INFORMATIONS POUR UN APPAREIL DE VENTILATION

(30) Priorität: 29.08.2013 DE 102013014303; 28.03.2014 DE 102014004448
(43) Veröffentlichungstag der Anmeldung: 06.07.2016
(73) Patentinhaber: Löwenstein Medical Technology S.A., 2557 Luxembourg (LU)
(72) Erfinder: FELDHAHN, Karl-Andreas, 22587 Hamburg (DE); SCHRÖTER, Christof, 76307 Karlsbad (DE); RENSMANN, Andreas, 76137 Karlsruhe (DE); STREMPEL, Uwe, 75177 Pforzheim (DE); SCHÄFER, Regina, 76187 Iffezheim (DE); SCHWAIBOLD, Matthias, 76228 Karlsruhe (DE)
(74) Vertreter: Marx, Thomas
(86) Internationale Anmeldenummer: PCT/DE2014/000453
(87) Internationale Veröffentlichungsnummer: WO 2015/027980

(56) Entgegenhaltungen:
- WO-A1-98/41267
- WO-A1-98/41267
- WO-A1-98/41267
- US-A- 6 158 432
- US-A- 6 158 432
- US-A- 6 158 432
- US-A1- 2008 072 896
- US-A1- 2008 072 896
- US-A1- 2008 072 896
- US-A1- 2011 138 308
- US-A1- 2011 138 308
- US-A1- 2011 138 308
- US-A1- 2012 291 783
- US-A1- 2012 291 783
- US-A1- 2013 069 896
- US-A1- 2013 069 896
- US-A1- 2013 104 886
- US-A1- 2013 104 886

## Beschreibung

Die Erfindung betrifft ein Bedien- und Informationssystem für ein Beatmungsgerät.

Die US 2012/291783 A1 offenbart ein Beatmungsgerät mit einem Anfeuchter. Das Beatmungsgerät ist zur Vorgabe der "high-flow therapy" (HFT) und zur Vorgabe der "continuous positive airway pressure" (CPAP) Therapie eingerichtet. Der Anfeuchter wird für beide Therapien unterschiedlich angesteuert. Es wird auch die Überwachung einer Schnittstelle zum Anfeuchter offenbart.

Die US 2013/104886 A1 offenbart einen Anfeuchter für ein Beatmungsgerät, bei dem die Erwärmung des Wassers gestoppt wird, wenn sensorisch ein Fehlen von Wasser angezeigt wird. Es wird auch die Kommunikation zwischen Beatmungsgerät und Anfeuchter offenbart.

Die US 2013069896 A1 offenbart eine tragbare elektronische Vorrichtung mit einem Touch-Display. Offenbart wird auch ein Energiesparmodus, bei dem das Display gedimmt oder verdunkelt wird. Wenn das Touch-Display in einen Energiesparmodus wechselt und ein Auslöseereignis erkannt wird, wird der Energiesparmodus beendet.

Die US 6158432 A offenbart ein Beatmungsgerät mit einem Bedien- und Informationssystem gemäß dem Oberbegriff des vorliegenden Hauptanspruchs.

Beatmungsgeräte weisen üblicherweise getrennte Bedien- und Informations- oder Anzeige-Elemente auf. Bedienelemente werden beispielsweise als Schalte oder Drehknöpfe ausgeführt. Die über die Bedienelemente erfolgten Einstellungen können dann an separaten Anzeigen abgelesen werden. Daraus resultiert eine komplexe Bedienung für den Anwender mit wenig intuitiven Menüführungen.

Aufgabe der vorliegenden Erfindung ist es daher, ein anwenderfreundliches und intuitiv bedienbares Bedien- und Informationssystem für ein Beatmungsgerät bereitzustellen, , welches eine einfache und intuitive Ankopplung und Bedienung eines Anfeuchters ermöglicht.

Die Aufgabe wird durch die Merkmale des Hauptanspruchs gelöst.

Das erfindungsgemäße Bedien- und Informationssystem für ein Beatmungsgerät, bevorzugt für ein CPAP, APAP, Bilevel oder Heimtherapie-Beatmungsgerät, ist mit einer Anzeige zur Darstellung von Informationen und zur Darstellung von Bedienfeldern für den Anwender und zumindest einem berührungsempfindlichen Eingabefeld in räumlicher Nähe zum dargestellten Bedienfeld ausgestattet wobei
- im Bereich der Anzeige ein erstes Bedienfeld und ein zweites Bedienfeld dargestellt werden,
- eine Verarbeitungseinheit, welche mit der Anzeige und der berührungsempfindlichen Eingabeeinheit gekoppelt ist, wobei die Verarbeitungseinheit ausgestaltet ist,
- eine Bedienung des ersten Bedienfeldes über die berührungsempfindliche Eingabeeinheit zu erfassen und in Abhängigkeit davon die Steuereinheit zu veranlassen die dem Bedienfeld zugeordnete Funktion auszuführen oder darzustellen.

Eine Bedienung des zweiten Bedienfeldes wird bevorzugt über die berührungsempfindliche Eingabeeinheit von der Verarbeitungseinheit erfasst wobei diese die Steuereinheit daraufhin veranlasst ein kontextabhängiges Untermenü aufzurufen, wobei das Untermenü eine Auswahl von betätigbaren Einstellfunktionen für das Menü umfasst, wobei die Steuereinheit eingestellte Parameter im Bereich des Menüs visualisiert und über Steuerbefehle an einen zugeordneten Aktor zur Anwendung bringt.

Der Steuereinheit ist ein Speicher zugeordnet und eingestellte Parameter oder Werte werden von der Steuereinheit in den Speicher geschrieben, wobei der Speicher zumindest die zuletzt eingegebenen und angewendeten Werte speichert.

Die Steuereinheit registriert Strom- oder Widerstands- oder Spannungsänderungen im Bereich der Schnittstellen oder der Anschlüsse zum Anfeuchter.

Die Steuereinheit erkennt über die Schnittstelle angeschlossene Speichermedien und ein angeschlossenes Speichermedium wird im Bereich der berührungsempfindlichen Eingabeeinheit als Bedienfeld darstellt und so können auch therapiefremde Daten in das Beatmungsgerät geladen werden und/oder von diesem ausgeführt werden.

Die Steuereinheit registriert Strom- oder Widerstands- oder Spannungsänderungen im Bereich der Schnittstellen oder der Anschlüsse zum Anfeuchter und erkennt so wenn ein Anfeuchter an den Anfeuchteranschluss adaptiert wird. Daraufhin wird von der Steuereinheit über die Anzeige ein neues Menü mit Bedienfeld für den Anfeuchter dargestellt.

Das Menü für die Anfeuchtersteuerung wird im Wesentlichen an einer bisher nicht mit einem Menü belegten Stelle der Anzeige dargestellt.

Benachbart zum Bedienfeld für den Anfeuchter werden zusätzliche Bedienfelder visualisiert werden, die zunächst nicht aktiv sind, wobei eine Betätigung des Bedienfeldes für den Anfeuchter von der Verarbeitungseinheit erfasst wird welche die Steuereinheit veranlasst die Bedienfelder zu aktivieren und wobei über diese eine Einstellung der Anfeuchterheizstufe erfolgt und die gewählte Anfeuchterstufe daraufhin im Bedienfeld angezeigt wird und zudem der gewählte Wert von der Steuereinheit über ein Steuersignal an Anfeuchter eingestellt und angewandt wird.

Die Steuereinheit registriert Strom- oder Widerstands- oder Spannungsänderungen im Bereich des Heizelementes des Anfeuchters und erkennt so einen sinkenden oder geringen Wasserstand anhand eines erhöhten Strom- oder Widerstands- oder Spannungswertes des Heizelementes. Dies veranlasst die Steuereinheit ein Symbol oder eine Textmeldung die einen niedrigen Wasserstand symbolisieren oder benennen im Bereich der Anzeige einzublenden.

Das Bedienfeld ist bevorzugt durch eine intensivere Farbe hervorgehoben ist, wodurch der Anwender auf die Einstellfunktion hingewiesen wird. Benachbart zum Bedienfeld können zusätzliche Bedienfelder in blasser Färbung visualisiert werden. Die blassere Farbe weist den Anwender darauf hin, dass die Felder nicht aktiv sind. Eine Betätigung des Bedienfeldes wird von der Verarbeitungseinheit erfasst welche die Steuereinheit veranlasst die zusätzlichen Bedienfelder in intensiver Färbung zu visualisieren. Zusätzlich können nun + und - Symbole visualisiert werden, über diese erfolgt eine Einstellung der Anfeuchterheizstufe. Die gewählte Anfeuchterstufe wird daraufhin im Bedienfeld angezeigt und zudem wird der geänderte Wert von der Steuereinheit über ein Steuersignal an Anfeuchter eingestellt und angewandt. Im Falle des Anfeuchters wird die Heizeinheit des Anfeuchters mit Wahl einer höheren Heizstufe aufgeheizt.

Erfindungsgemäß ist immer eine Bedienfläche oder ein Bedienelement vorgesehen (Grundzustand), dessen Betätigung das Bedien- und Informationssystem veranlasst über die Steuereinheit den Grundzustand darzustellen. Dazu werden aktive Bedienfelder oder andere Inhalte ausgeblendet.

Das Bedien- und Informationssystem ist auch dadurch gekennzeichnet, dass das Bediensystem eine grafische Einstellhilfe für mindestens eine Rampensteilheit umfasst.

Das Bedien- und Informationssystem ist auch dadurch gekennzeichnet, dass eine grafische Einstellhilfe für die Triggersensitivität vorhanden ist.

Das Bedien- und Informationssystem ist auch dadurch gekennzeichnet, dass mindestens ein Einstellelement als ein Drehrad ausgebildet ist.

Das Bedien- und Informationssystem ist auch dadurch gekennzeichnet, dass mindestens ein kreisförmiges Anzeigeelement verwendet ist.

Das Bedien- und Informationssystem ist auch dadurch gekennzeichnet, dass ein Dreh-Drückknopf verwendet ist.

Das Bedien- und Informationssystem ist auch dadurch gekennzeichnet, dass eine Start-Stoppfläche verwendet ist.

Das Bedien- und Informationssystem ist auch dadurch gekennzeichnet, dass die Start-Stoppbedienfläche im Bereich des Touchscreens angeordnet ist.

Das Bedien- und Informationssystem ist auch dadurch gekennzeichnet, dass die Start-Stoppbedienfläche von einer Gerätesteuerung in unterschiedlichen Gestaltungen generierbar ist.

Das Bedien- und Informationssystem ist auch dadurch gekennzeichnet, dass drei feste Stufen für den Beatmungsparameter vorgesehen sind und diese Stufen sind mittels der Symbole +/-verstellbar sind, um so den Beatmungsparameter patientenindividuell feiner abzustimmen.

Die erfindungsgemäße Bedienvorrichtung für ein Beatmungsgerät, umfasst
a) eine grafische Anzeige die zumindest zeitweise den Wertebereich für einen Beatmungsparameter repräsentiert und zumindest einzelne Werte numerisch darstellt, einen Speicher für Beatmungsparameter-Werte
b) mindestens einen mit dem Wertebereich verbundenen Datenpunkt mindestens eine mit dem Datenpunkt schaltlogisch verbundene Stelle der grafischen Anzeige
c) eine Schaltlogik, die bei Betätigung der, dem Datenpunkt schaltlogisch zugeordneten Stelle des mechanischen Bedienelementes
d) eine Anzeige von mindestens einem, dem Datenpunkt zugeordneten, Zahlenwert und /oder eines Bestätigungsfeldes für den Zahlenwert veranlasst
e) eine Schaltlogik, die bei Betätigung der, dem Datenpunkt schaltlogisch zugeordneten Stelle des mechanischen Bedienelementes diesen Zahlenwert auf den zugeordneten Atemgasparameter anwendet und diesen Zahlenwert mit dem zugeordneten Atemgasparameter in den Speicher schreibt.

Das Bedien- und Informationssystem ist auch dadurch gekennzeichnet, dass kein berührungsempfindliches Anzeigeelement verwendet wird, sondern nur ein grafisches Anzeigeelement mit einem separat ausgeführten mechanischen Bedienelement und die mittels des mechanischen Bedienelementes vorgenommen Einstellungen auf dem grafischen Anzeigeelement graphisch visualisiert werden.

Das Bedien- und Informationssystem ist auch dadurch gekennzeichnet, dass auf dem grafischen Anzeigeelement die Veränderung der Werte während des Einstellvorganges über das mechanische Bedienelement und/oder der ausgewählte Werte und/oder der zur Verfügung stehenden Wertebereiches visualisiert werden.

Erfindungsgemäß ist vorgesehen, dass ein aktives Bedienfeld durch eine intensivere Farbe hervorgehoben ist, wodurch der Anwender auf die Einstellfunktion hingewiesen wird und nicht aktive Bedienfelder in blasser Färbung visualisiert werden. Die blassere Farbe weist den Anwender darauf hin, dass die Felder nicht aktiv sind.

Durch größtenteils optisch umgesetzte Rückmeldungen verschiedenster Art, kommuniziert das erfindungsgemäße Gerät mit dem Bediener. Im Hinblick auf mögliche Benutzungsfehler ist durch beispielsweise durch Tasten- Feedback eine Selbstkontrolle des Bedieners möglich. Das erhöht die Sicherheit im Umgang mit dem Gerät, reduziert die Gefährdungen durch falsch vorgenommene Einstellungen.

### Formen des Feedbacks:

Symbolik für Erfolg Die Maskendichtigkeit wird über grüne Quadrate visualisiert. Die Nutzungsdauer wird über grüne Herzen visualisiert, sofern Sie innerhalb der Vorgaben liegt. Wenn die Nutzungsdauer zu gering ist, die Nutzungsdauer abgestuft in Orange oder Rot visualisiert.

Farbschema Darstellung Einstellwerte werden weiß oder schwarz dargestellt. Messwerte die im definierten Bereich liegen werden grün dargestellt. Kritische Werte werden rot dargestellt. Warnungen werden orange dargestellt.

Blinkende Rückmeldung Ein aktiver, noch nicht abgeschlossener Vorgang wie beispielsweise das Speichern von Daten oder die Fernübertragung von Daten wird durch Blinken visualisiert.

In den Zeichnungen sind Ausführungsbeispiele der Erfindung schematisch dargestellt. Es zeigen:
- Fig. 1: eine perspektivische Darstellung eines Beatmungsgerätes mit Atemmaske und Atemgasschlauch,
- Fig. 2: eine Anzeige des Bedien- und Informationssystems,
- Fig. 3: eine Anzeige zur Veranschaulichung eines Untermenüs,
- Fig. 4: eine Anzeige zur Veranschaulichung, wie das Bedien- und Informationssystem einen angeschlossenen Anfeuchter erkennt und ansteuert,
- Fig. 5: eine Darstellung ähnlich zu Figur 4 in einem anderen Betriebszustand,
- Fig. 6: eine Darstellung ähnlich zu Figur 4 und Figur 5 zur Veranschaulichung eines weiteren Betriebszustandes,
- Fig. 7: eine schematische Darstellung einer grafischen Einstellhilfe für eine Rampensteilheit,
- Fig. 8: eine schematische Darstellung einer grafischen Einstellhilfe für die Trigger-Sensitivität,
- Fig. 9: eine grafische Einstellhilfe für mindestens einen Druck,
- Fig. 10: eine ergänzende Darstellung zu Figur 7,
- Fig. 11: eine Abbildung zur Veranschaulichung eines Verhältnisses von inspiratorischer Zeit sowie der gesamten Atemzugszeit und
- Fig. 12: eine schematische Darstellung eines kreisförmigen Bedienelementes,
- Fig. 13: eine alternative Ausführungsform und
- Fig. 14: ein mechanisches Bedienelement.

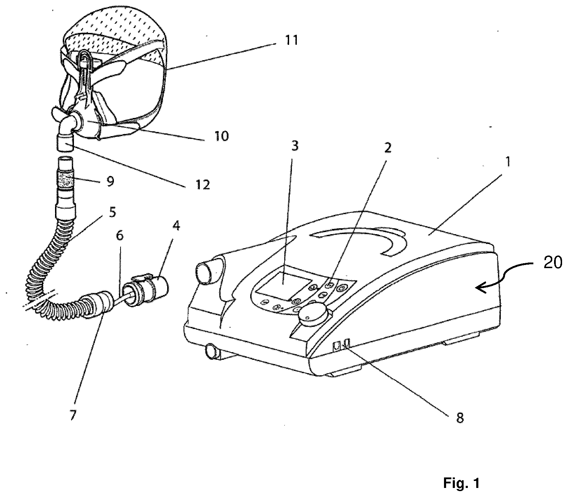

Fig. 1 zeigt den grundsätzlichen Aufbau einer Vorrichtung zur Beatmung. Im Bereich eines Gerätegehäuses (1) des Beatmungsgerätes (20), mit einer Atemgasquelle im Geräteinnenraum, sind ein Bedienelement (2) und ein Bedien-und Informationssystem (3) bestehend aus einer Anzeige (13), einer berührungsempfindlichen Eingabeeinheit (15) mit zumindest einem Bedienfeld (14) angeordnet. Über eine Kopplung (4) wird ein Verbindungsschlauch (5) angeschlossen. Entlang des Verbindungsschlauches (5) kann ein zusätzlicher Druckmessschlauch (6) verlaufen, der über einen Druckeingangsstutzen (7) mit dem Gerätegehäuse (1) verbindbar ist. Zur Ermöglichung einer Datenübertragung weist das Gerätegehäuse (1) zumindest eine Schnittstelle (8) auf. Ein Anfeuchter kann zudem adaptiert werden.

Im Bereich einer dem Gerätegehäuse (1) abgewandten Ausdehnung des Verbindungsschlauches (5) ist ein Ausatmungselement (9) angeordnet. Ebenfalls kann ein Ausatemventil verwendet werden.

Fig. 1 zeigt darüber hinaus ein als Beatmungsmaske (10) ausgebildetes Patienten-Interface, das als Nasalmaske realisiert ist. Eine Fixierung im Bereich eines Kopfes eines Patienten kann über eine Kopfhaube (11) erfolgen. Im Bereich seiner dem Verbindungsschlauch (5) zugewandten Ausdehnung weist das Patienten-Interface (10) ein Kupplungselement (12) auf.

Über die Schnittstelle (8) kann die Eingabe und / oder Ausgabe von Daten, wie beispielsweise Totraumvolumen, erfolgen. Die Schnittstellen können kabelgebunden, als Infrarot-Schnittstelle, als Bluetooth-Schnittstelle oder USB realisiert sein. Bevorzugt ist auch ein Kartenschacht vorgesehen. Die Schnittstelle (8) kann auch als LAN-Schnittstelle oder als sonstige Schnittstelle zur Anbindung an das Internet ausgeführt sein. Im Bereich eines Gerätegehäuses kann ein Sauerstoffzuschaltventil an die Vorrichtung zur Beatmung adaptiert werden. Es ist denkbar, das Atemgas zusätzlich mit Sauerstoff anzureichern, um die Patientenversorgung zu verbessern.

Über die Schnittstelle (8) - beispielsweise als Kartenschacht oder USB ausgeführt - können auch therapiefremde Daten in das erfindungsgemäße Beatmungsgerät geladen werden beziehungsweise von diesem ausgeführt werden. So ist daran gedacht beispielsweise Fotos oder Videos mittels Speichermedien über die Schnittstelle (8) im Bereich der Anzeige darzustellen. Der Anwender muss - werden vom Gerät externe Speichermedien erkannt - eine Abfrage im Bedienfeld bestätigen, woraufhin die Daten wahlweise im Bereich des Beatmungsgerätes gespeichert oder ausgeführt werden.

Das erfindungsgemäße Beatmungsgerät (20) ist so ausgelegt, dass es über einen Schlauch und ein Patienteninterface mit einem Patienten verbunden werden kann, um eine Beatmung bereitzustellen. Es umfasst eine Quelle für Atemgas, die beispielsweise als ein Elektromotor mit Gebläserad ausgebildet ist, und eine Einrichtung zur Ermittlung von Druck und/oder Fluss und/oder Volumen des Atemgases, sowie eine Steuereinheit , die so ausgelegt ist, dass sie für jeden Atemzyklus auf der Basis eines vorbestimmten Wertes für den Patienten und/oder auf Basis von Messsignalen für die Parameter Druck und/oder Flow und/oder Volumen einen Atemgasdruck bestimmt und die Quelle für Atemgas derart reguliert, dass der Atemgasdruck erzeugt wird.

Die Steuereinheit ist ferner so ausgelegt, dass sie den aktuellen Druck und/oder Flow und/oder das Volumen von Atemgas bestimmt und den aktuellen Wert über das mit der Steuereinheit Bedien- und Informationssystem (3) darstellt. Die Steuereinheit ist außerdem so ausgelegt, dass sie bezogen auf einen oder mehrere Parameter Trendveränderungen ihrer Berechnungen über der Zeit bestimmt, wobei die Trendveränderungen auf der Anzeige angezeigt werden können.

Weiterhin vergleicht die Steuereinheit solche Parameter-Werte, die von einem Benutzer vorgegeben wurden, beispielsweise obere und untere Druckgrenzen oder eine maximal tolerierbare Anzahl von Apnoen pro Zeiteinheit, oder eine maximal tolerierbare Leckage, mit den aktuellen Werten und generiert eine Benutzer-Information zu Abweichungen von der Vorgabe. Die Benutzerinformation wird bevorzugt über das Bedien-und Informationssystem (3) graphisch visualisiert.

So werden aus dem gemessenen Atemfluss über eine Abnahme des Atem(zeit)volumens für eine Zeitdauer von mindestens 10 s Apnoen und Hypopnoen erkannt. Zusätzlich wird über Druck- und Flowschwankungen Schnarchen erkannt, sowie über die inspiratorische Flowkontur Flattening erkannt. Daraus werden für jede ausreichend lange nächtliche Therapie Indizes berechnet, nämlich: AHI (= Anzahl Apnoen + Hypopnoen pro artefaktfreie Therapiedauer), RDI (= Anzahl aller respiratorischen Ereignisse pro artefaktfreie Therapiedauer), Anteil Atemzüge mit Flattening, Anteil Atemzüge mit Schnarchen. Bevorzugt werden auch Daten ermittelt, die auf das Nutzungsverhalten oder auf die Nutzungsdauer des Gerätes durch den Patienten schließen lassen. Diese Daten werden täglich oder wöchentlich oder monatlich ermittelt und gespeichert. Bedarfsweise werden die Nutzungsdaten, gegebenenfalls zusammen mit einer Gerätekennung, über eine Internetverbindung oder Mobilfunkverbindung abgerufen und versendet.

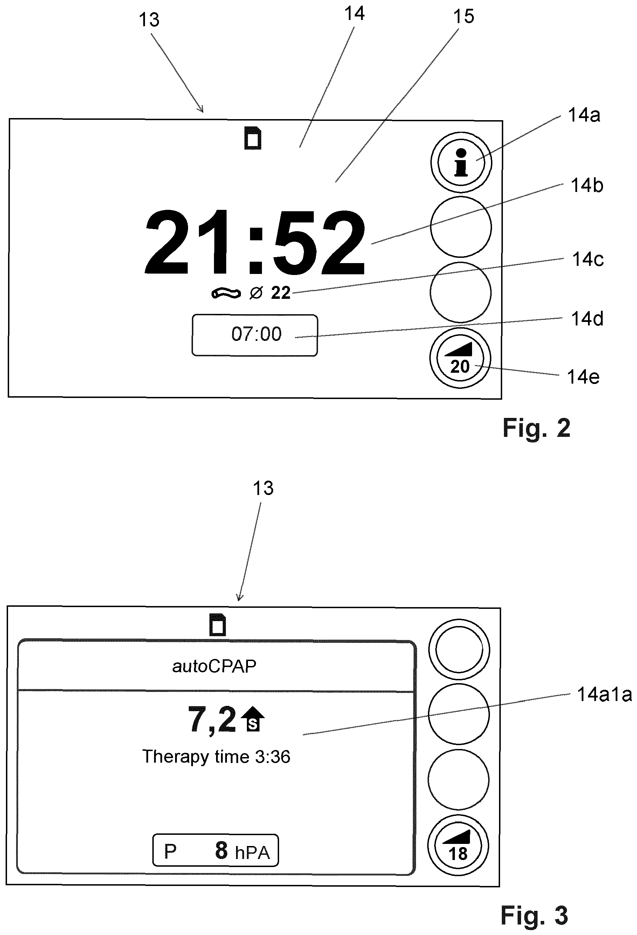

Figur 2 zeigt das Bedien- und Informationssystem (3) für ein Beatmungsgerät (20) mit einer beleuchteten oder hinterleuchteten Anzeige (13) zur Darstellung von Bedienfeldern (14) oder Informationen für den Anwender und einer berührungsempfindlichen Eingabeeinheit (15) in räumlicher Nähe zum dargestellten Bedienfeld (14). In einer konkreten Ausführungsform handelt es sich um eine Mensch-Maschine-Schnittstelle in Form eines sogenannten Touchscreens, wobei der Fachmann unterschiedliche Typen kennt die alle als Bestandteil des erfindungsgemäßen Bedien- und Informationssystem (3) in Frage kommen. Im Bereich der Anzeige werden zumindest ein erstes Bedienfeld (14a) und ein zweites Bedienfeld (14b) dargestellt. Die Steuereinheit ist ausgestaltet das Menü auf der Anzeige (13) darzustellen.

Eine Verarbeitungseinheit welche mit der Anzeige (13) und der berührungsempfindlichen Eingabeeinheit (15) gekoppelt ist, ist ausgestaltet eine Bedienung des Bedienfeldes (14) über die Eingabeeinheit (15) zu erfassen und in Abhängigkeit davon eine Funktion des Menüs über die Steuereinheit anzusteuern. Bevorzugt wir ein, einem Bedienfeld (14b, 14c, 14d ...) zugeordnetes, Menü auf der Anzeige (13) räumlich benachbart zum Bedienfeld oder an derselben Stelle des Bedienfeldes dargestellt. Eine zeitgleiche oder zeitversetzte Bedienung weiterer Bedienfelder (14b, 14c, 14d ...) über die Eingabeeinheit (15) kann ebenfalls über die Verarbeitungseinheit erfasst werden. Die Verarbeitungseinheit veranlasst daraufhin die Steuereinheit, das dem gewählten Bedienfeld zugeordnete Menü in der ersten Ebene (=Untermenü) aufzurufen. Bevorzugt wir ein, einem Bedienfeld (14b, 14c, 14d ...) zugeordnetes, Untermenü auf der Anzeige (13) räumlich benachbart zum Bedienfeld oder zum Menü oder an derselben Stelle des Bedienfeldes / Menüs dargestellt.

Von der Steuereinheit wird über die Anzeige (13) ein Untermenü dargestellt. Bevorzugt wird das Untermenü im Wesentlich an derselben Stelle dargestellt, wie das Menü, aus dem Untermenü hervorgeht. Im Untermenü können nun weitere Bedienfelder (14) oder Informationen dargestellt werden. Grundsätzlich ist eine Navigation in mehrere Untermenüs vorgesehen; bevorzugt ist die Verzweigung jedoch nicht tiefer als zwei Menüebenen. Um aus einem Untermenü wieder in das Menü zurückzukehren, ist immer an derselben Stelle ein Bedienfeld (14) vorgesehen, dessen Betätigung die Steuereinheit veranlasst auf der Anzeige (13) das Menü der nächsthöheren Hierarchiestufe darzustellen.

Die Steuereinheit weist auch eine Uhr auf, die die Zeit ermittelt und im Bereich der Anzeige darstellt. Gekoppelt damit ist auch eine Weckzeit/Alarmzeit mit einer Signaleinrichtung. Der Nutzer kann eine Zeit vorbestimmen, bei deren Erreichen über die Signaleinrichtung ein akustischer und/oder optischer Alarm ausgegeben wird.

In der Grunddarstellung weist die Anzeige (13) zentral die aktuelle Uhrzeit aus. Darunter wird optional die eingegebene Weckzeit dargestellt. Uhrzeit und Weckzeit lassen sich durch Berührung der jeweils zugeordneten Bedienfelder (14b, 14d) einstellen. Die Bedienfelder sind bevorzugt an der Stelle, an der auch die Uhrzeit bzw. die Weckzeit angezeigt werden.

Zwischen Uhrzeit und Weckzeit wird der durchschnittliche Therapiedruck angezeigt. Zudem ist an dieser Stelle auch ein Bedienfeld (14c) vorgesehen, über welches man in das Untermenü zum durchschnittlichen Therapiedruck gelangt.

In der unteren rechten Ecke ist der aktuell eingestellte Beatmungsdruck in hPa, mit zugeordnetem Bedienfeld (14e), dargestellt. In der oberen rechten Ecke ist ein Informationsfeld mit zugeordnetem Bedienfeld (14a) dargestellt.

Figur 3 veranschaulicht das Untermenü zum Bedienfeld (14a =Informationsfeld). Eine Betätigung des Informationsfeldes (14a) wird von der Verarbeitungseinheit erfasst. Die Verarbeitungseinheit veranlasst daraufhin die Steuereinheit, das dem Informationsfeld (14a) zugeordnete Menü (Informationsmenü) aufzurufen und darzustellen. Die Darstellung erfolgt bevorzugt mittig im Bereich der Anzeige (3), wobei die Anzeige von Uhr (14b) und Weckzeit (14d) gleichzeitig ausgeblendet wird. Die Anzeige des Informationsmenüs erfolgt zeitgesteuert. Die Steuereinheit wechselt nach einer Anzahl von Sekunden wieder zur Grunddarstellung zurück. Beispielsweise nach fünf Sekunden. Dazu weist die Steuereinheit eine Countdown-Einheit auf, die die fünf Sekunden zählt und dann die Steuereinheit veranlasst wieder die Grunddarstellung mit Uhr und Weckzeit zu aktivieren.

Im Bereich des Informationsmenüs erfolgt die Anzeige von Werten zum Therapieverlauf, nämlich beispielsweise AHI, Compliance, Nutzungsdauer, Leckage und optional weiteren Werten. Eine Anwahl der dargestellten Werte öffnet ein neues Untermenü, in dem weitere Details und Einstellmöglichkeiten zu dem angezeigten Wert veranschaulicht werden.

Grundsätzlich ist vorgesehen betätigbare Einstellfunktionen - wie die Anfeuchterstufe, den Therapiedruck, die Uhrzeit, die Weckzeit oder weitere - graphisch, beispielsweise durch unterschiedliche Farbe oder unterschiedliche Intensität der Farbe - hervorzuheben, damit der Anwender sofort erkennt, welche angezeigte Information einstellbar ist. Im Feld der betätigbaren Einstellfunktionen wird zusätzlich der aktuell eingestellt Wert visualisiert. Wenn der Anwender eine solche betätigbare Einstellfunktion anwählt wird von der Steuereinheit ein ergänzendes zugeordnetes Menüfeld - das Bedienfeld - auf der Anzeige und benachbart zur gewählten betätigbaren Einstellfunktion dargestellt. Im einfachsten Fall werden oberhalb und unterhalb oder rechts und links von der gewählten betätigbaren Einstellfunktion zwei Bedienfelder in Form von + und - Symbolen visualisiert. Eine Betätigung der Bedienfelder wird von der Verarbeitungseinheit erfasst, welche daraufhin die Steuereinheit veranlasst den Wert der betätigbaren Einstellfunktionen entsprechend der Eingabe zu verändern und im Bereich der Anzeige (3) zu visualisieren. Dabei ist vorgesehen, dass die Steuereinheit den Wert nicht nur visualisiert, sondern auch den zugeordneten Aktor per Steuerbefehl zur Anpassung oder Anwendung des Wertes veranlasst. Bevorzugt werden zunächst der Einstellwert und der Ist-Wert visualisiert und nachdem der Ist-Wert dem Einstellwert entspricht, wird nur noch der Ist-Wert dargestellt.

Wenn beispielsweise der Beatmungs-Druck - als betätigbare Einstellfunktionen - geändert werden soll, berührt der Anwender einfach das entsprechende Feld der Anzeige, in dem die Information über den aktuellen Druck angezeigt wird, hier (14e). Daraufhin wird von der Steuereinheit die Anzeige eines Bedienfeldes veranlasst. Das Bedienfeld kann eine rechnerähnliche Tastatur sein die auf einem überlagerten Fenster visualisiert wird. Auf dieser Tastatur kann der Anwender den neuen Wert für den gewählten Parameter eingeben. Nachdem der gewünschte Parameter eingegeben wurde, bestätigt der Anwender den Wert durch ein angezeigtes ENTER-Symbol woraufhin die Rechneranzeige wieder verschwindet. Im einfachsten Fall werden oberhalb und unterhalb oder rechts und links von der gewählten betätigbaren Einstellfunktion zwei Bedienfelder in Form von + und - Symbolen visualisiert. Eine Betätigung der Bedienfelder wird von der Verarbeitungseinheit erfasst, welche daraufhin die Steuereinheit veranlasst den Wert der betätigbaren Einstellfunktionen entsprechend der Eingabe zu verändern und im Bereich der Anzeige zu visualisieren. Der geänderte Parameter wird von der Steuereinheit in dem entsprechenden Feld der Anzeige dargestellt, und gleichzeitig oder erst nach Anwenderauswahl über ein Steuersignal an das Gebläse des Beatmungsgerätes als neuer Parameter eingestellt und angewandt. Bevorzugt werden zunächst der Einstellwert und der Ist-Wert visualisiert und nachdem der Ist-Wert dem Einstellwert entspricht, wird nur noch der Ist-Wert dargestellt.

Die so eingestellten Parameter werden von der Steuereinheit gleichzeitig in einen Speicher geschrieben, welcher als Zwischenspeicher für die aktuell anzuwendenden Parameterwerte dient.

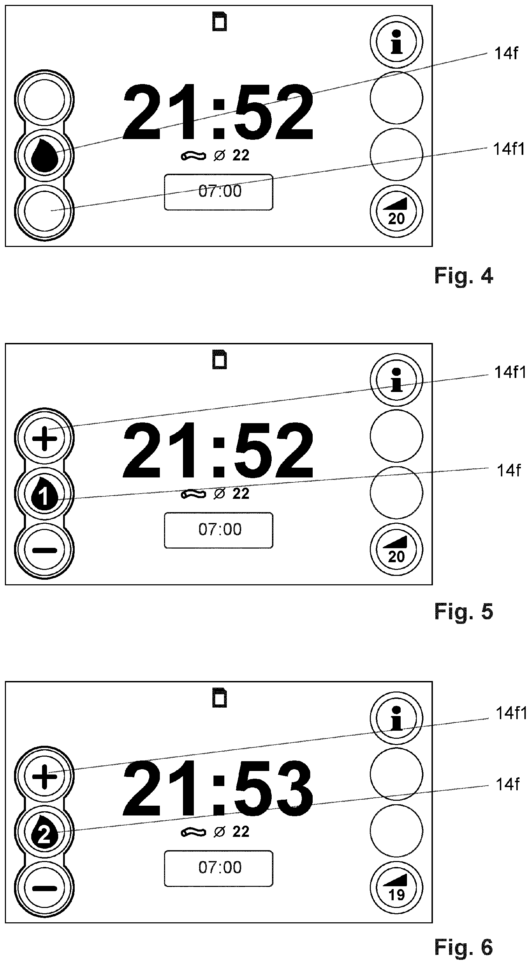

Die Figuren 4 bis 6 veranschaulichen nun, wie über das erfindungsgemäße Bedien-und Informationssystem ein angeschlossener Anfeuchter erkannt und gesteuert wird. Die Steuereinheit registriert Strom- oder Widerstands- oder Spannungsänderungen im Bereich der Schnittstellen oder der Anschlüsse zum Anfeuchter. Wird nun ein Anfeuchter an den Anfeuchteranschluss adaptiert, so wird dies von der Steuereinheit registriert. Daraufhin wird von der Steuereinheit über die Anzeige (13) ein neues Menü mit Bedienfeld für den Anfeuchter (14f) dargestellt. Bevorzugt wird das Menü für die Anfeuchtersteuerung im Wesentlichen an einer bisher nicht mit einem Menü belegten Stelle der Anzeige dargestellt.

Das Bedienfeld (14f) ist durch eine intensivere Farbe hervorgehoben, wodurch der Anwender auf die Einstellfunktion hingewiesen wird. Benachbart zum Bedienfeld (14f) werden zusätzlich zwei Bedienfelder (14f1) in blasser Färbung visualisiert. Die blassere Farbe weist den Anwender darauf hin, dass die Felder nicht aktiv sind. Eine Betätigung des Bedienfeldes (14f) wird von der Verarbeitungseinheit erfasst welche die Steuereinheit veranlasst die zwei Bedienfelder (14f1) in intensiver Färbung zu visualisieren. Zusätzlich werden nun + und - Symbole visualisiert. Über diese erfolgt eine Einstellung der Anfeuchterstufe. Die gewählte Anfeuchterstufe wird daraufhin im Bedienfeld (14f) angezeigt und zudem wird der geänderte Wert von der Steuereinheit über ein Steuersignal an Anfeuchter eingestellt und angewandt. Im Falle des Anfeuchters wird die Heizeinheit des Anfeuchters mit Wahl einer höheren Heizstufe aufgeheizt.

Die Steuereinheit registriert Strom- oder Widerstands- oder Spannungsänderungen im Bereich des Heizelementes des Anfeuchters. Bei sinkendem Wasserstand erhöht sich der Widerstand des Heizelementes. Dies wird von der Steuereinheit erfasst. Daraufhin blendet die Steuereinheit ein Symbol für einen niedrigen Wasserstand im Bereich der Anzeige ein oder gibt eine entsprechende Textmeldung aus.

Grundsätzlich ist vorgesehen, dass eine Bedienung eines zweiten Bedienfeldes über die berührungsempfindliche Eingabeeinheit von der Verarbeitungseinheit erfasst wird und die Steuereinheit daraufhin veranlasst wird ein kontextabhängiges Untermenü, beispielsweise in Form zusätzlicher Bedienfelder, aufzurufen, wobei das Untermenü eine Auswahl von betätigbaren Einstellfunktionen, beispielsweise in Form von Plus- und Minus-Tasten um einen Einstellwert anzupassen, für das Menü umfasst und wobei die Steuereinheit eingestellte Parameter im Bereich des Menüs visualisiert und über Steuerbefehle an einen zugeordneten Aktor zur Anwendung bringt.

Grundsätzlich ist vorgesehen, Bedienvorgänge und Geräteausgaben zu priorisieren. Dies erfolgt entsprechend hinterlegter Regeln automatisch durch die Steuereinheit. Wird beispielsweise ein höher priorisierter Bedienvorgang oder eine Geräteausgabe wie ein Warnhinweis aktiv, so wird ein aktuelles Bedienfeld (14x) durch den höher priorisierten Bedienvorgang oder die Geräteausgaben überblendet. Dabei stellt die Steuereinheit das, dem höher priorisierten Bedienvorgang oder der Geräteausgabe, zugeordnete Bedienfeld unmittelbar im Bereich der Anzeige (13) in den Vordergrund und das aktuelle Bedienfeld (14x) tritt in den Hintergrund.

Die Steuereinheit des erfindungsgemäße Bedien- und Informationssystem registriert Strom- oder Widerstands- oder Spannungsänderungen im Bereich der Schnittstellen oder der Anschlüsse zum Anfeuchter. Wird nun eine Speicherkarte in oder ein USB-Stick in die entsprechende Schnittstelle gesteckt, so wird dies von der Steuereinheit registriert. Daraufhin wird von der Steuereinheit über die Anzeige (13) ein neues Menü mit Bedienfeld für das Speichermedium (14s) dargestellt. Bevorzugt wird das Menü für das Speichermedium (14s) im Wesentlichen an einer bisher nicht mit einem Menü belegten Stelle der Anzeige dargestellt.

Alternativ ist auch daran gedacht das Menü (14s) zentral im Bereich der Anzeige darzustellen. Das zugeordnete Bedienfeld (14s1) wird benachbart zum Menü dargestellt und ist beispielsweise durch eine intensivere Farbe hervorgehoben, wodurch der Anwender auf die Einstellfunktion hingewiesen wird. Eine Betätigung des Bedienfeldes (14s1) wird von der Verarbeitungseinheit erfasst welche die Steuereinheit veranlasst die Inhalte des Speichermediums darzustellen oder auszuführen. Es ist erfindungsgemäß daran gedacht, über einen USB-Stick oder eine Speicherkarte Fotos oder Videos auf der Anzeige darstellen zu lassen. Dazu wird im zugeordneten Bedienfeld (14s1) abgefragt, ob die Fotos oder Videos dargestellt werden sollen.

Eine Berührung des Bedienfeldes (14s1) wird von der Verarbeitungseinheit erfasst, welche die Steuereinheit veranlasst alle bisher im Bereich der Anzeige dargestellten Informationen oder Menüs auszublenden und die Fotos oder Videos von dem Speichermedium im Bereich der Anzeige im Wesentlichen großflächig darzustellen. Bevorzugt erfolgt eine Darstellung von Fotos automatisch sequenziell mit wählbarer Einblendzeit.

Sofern während der Darstellung der Fotos Alarme entstehen, werden diese von der Steuereinheit höher Priorisiert und statt der Fotos dargestellt. Sofern der Anwender Funktionen des Beatmungsgerätes oder des Anfeuchters überprüfen oder einstellen will, so berührt er während der Fotodarstellung die berührungsempfindliche Eingabeeinheit (15), an einer beliebigen Stelle. Die Verarbeitungseinheit erfasst dies und veranlasst die Steuereinheit die Fotodarstellung auszublenden und die Grunddarstellung des Beatmungsgerätes gemäß Figur 2 darzustellen. Nach Vornahme der gewünschten Einstellungen durch den Anwender, wechselt die Steuereinheit automatisch wieder zur Darstellung der Fotos zurück.

Erfindungsgemäß sind ein Arzt und ein Patienten-Menü vorgesehen. Das Arzt-Menü bietet alle wichtigen Einstellmöglichkeiten während das Patientenmenü lediglich reduzierte Einstellmöglichkeiten bietet. Dazu ist eine Freigabefunktion für den Arzt vorgesehen. Zur Freigabe des Arztmenüs muss der Arzt beispielsweise eine Kennung eingeben oder sich über eine Chipkarte authentifizieren. Erst dann werden alle Einstellmöglichkeiten freigeschaltet. Bestimmte Bedienelemente sind nur dann aktiviert und sichtbar, wenn eine erfolgreiche Authentifizierung erfolgt ist, beispielsweise diejenigen zum Management von Alarmen: Aktivierung/Deaktivierung, Bestätigung/Quittierung, Wahl des Alarm-Schwellwertes.

Der Patient wird nicht durch Bedienelemente verunsichert, die für ihn zwar sichtbar, aber gesperrt sind. Im Patienten-Menü werden bevorzugt therapiefremde Informationen und Einstellmöglichkeiten im Grundzustand dargestellt. So erfolgt die Anzeige von Uhrzeit, Weckzeit zentral im Bereich der Anzeige. Im Arzt-Menü erfolgt bevorzugt die Anzeige therapierelevanter Informationen - Visualisierung und Bedienung von Beatmungsparametern - im Bereich der Anzeige. Bevorzugt an derselben Stelle wie die Uhrzeit und Weckzeit im Patienten-Menü.

### Beispiel Rampenfunktion:

Die Rampenfunktion bietet dem Patienten zunächst einen geringeren Druck als den Therapiedruck, der im Laufe von Minuten auf den Therapiedruck angehoben wird. Der Vorteil für den Patienten ist, dass das Einschlafen erleichtert wird. Wacht der Patient nachts auf und möchte anschließend wieder mit der Rampenfunktion einschlafen, so kann er erfindungsgemäß die Rampenfunktion durch eine Berührung des erfindungsgemäße Bedien- und Informationssystem auslösen. Dies kann bevorzugt im Halbschlaf oder bei schlechter Sicht in Dunkelheit oder ohne eine Brille dadurch erfolgen, dass das erfindungsgemäße Bedien- und Informationssystem die Bedienfläche (14) für die Rampenfunktion derart vergrößert oder positioniert, dass sie bevorzugt immer bei Berührung des Bedien- und Informationssystem aktiviert wird. Dazu wird die Rampenfunktion von der Steuereinheit zeitgesteuert immer dann aktiviert, wenn über eine definierte Zeitdauer keine Benutzung des Bedien- und Informationssystem festgestellt wurde. In anderen Bediensituationen während der Patient wach ist, hat die Rampenfunktion dagegen keinerlei Bedeutung. Die Schaltfläche wird dann wieder ausgeblendet oder verkleinert und wichtigere Funktionen rücken in den Vordergrund.

Je nach Art des Parameters ist ein anderes Einstellprinzip sinnvoll. Das erfindungsgemäße Bedien- und Informationssystem (3) ermöglicht es, für jeden Parameter die effizienteste Einstellmöglichkeit anzubieten. Beispielsweise kann zur Eingabe von Zahlencodes, IP-Adressen, Datum, Seriennummer, oder Buchstaben das Bedienfeld als ein Ziffernblock und/oder Buchstabenblock erfolgen. Für die Verstellung von Parametern mit wenigen definierten Einstellstufen kann das Bedienfeld als +/- Feld oder Stufenanzeige erfolgen.

Ein Bedienfeld in Form eines Zahlenstrahls kann für die Einstellung von Parametern mit vielen Einstellstufen, wie beispielsweise den Beatmungsdruck oder die Hintergrundfrequenz von 6 - 40 1/min, erfolgen.

Ein Bedienfeld in Form einer Listenauswahl wird bei der Einstellung vieler Optionen eingeblendet, wie beispielsweise zur Auswahl der Sprache für das Userinterface. Wobei die auswählbaren Optionen in Schriftform in einer Liste angezeigt werden und die Auswahl der gewünschten Option durch Berührung derselben erfolgt, woraufhin die Steuereinheit über ein Steuersignal die Umsetzung der Auswahl veranlasst.

Ein Bedienfeld in Form Blättertasten dient zur Umschaltung ganzer Bildschirminhalte. Hierzu werden rechts und links im Bedien- und Informationssystem Bedienflächen mit beispielsweise Pfeilsymbolen eingeblendet, deren Berührung die Steuereinheit veranlasst über ein Steuersignal den nächsten Bildschirminhalt darzustellen.

Erfindungsgemäß ist immer eine Bedienfläche (14g) oder ein Bedienelement (2) vorgesehen (Grundzustand), dessen Betätigung das Bedien- und Informationssystem (3) veranlasst über die Steuereinheit den Grundzustand darzustellen. Dazu werden aktive Bedienfelder (14) oder andere Inhalte ausgeblendet.

Erfindungsgemäß ist vorgesehen, dass ein aktives Bedienfeld (14) durch eine intensivere Farbe hervorgehoben ist, wodurch der Anwender auf die Einstellfunktion hingewiesen wird und nicht aktive Bedienfelder in blasser Färbung visualisiert werden. Die blassere Farbe weist den Anwender darauf hin, dass die Felder nicht aktiv sind.

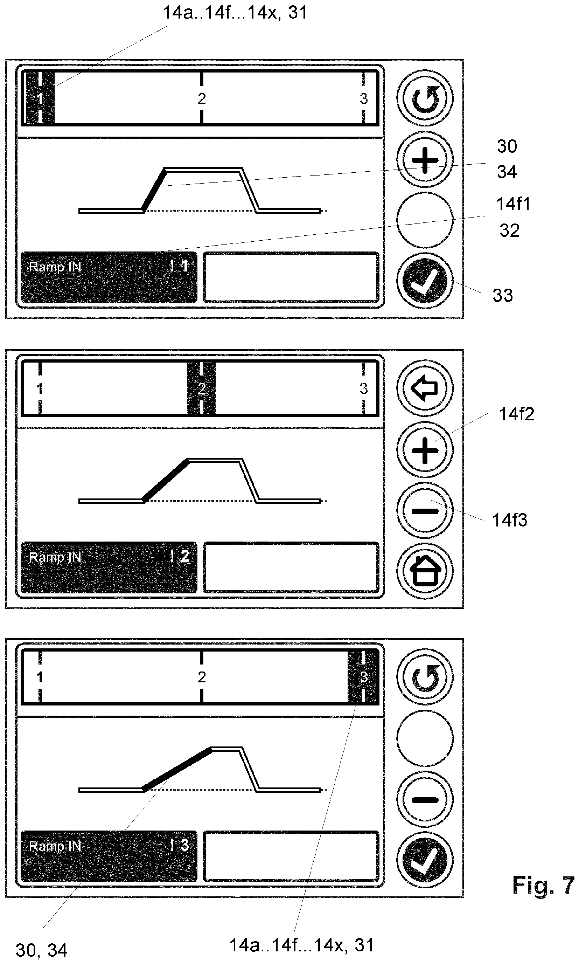

Die Abbildung 7 zeigt die grafische Einstellhilfe für die Rampensteilheit für den Übergang von exspiratorischem Druck auf inspiratorischen Druck (grün markiert). Die aktuelle Stufe kann per Slider/Lineal oder alternativ mit + und - eingestellt werden. Das Lineal dient zusätzlich mit seiner grünen Markierung nicht nur als Einstellwerkzeug, sondern auch als Anzeigeelement. Dies bietet den Vorteil, dass auch ungeübte Nutzer direkt bei der Einstellung den Wert angezeigt bekommen.

Im dargestellten Beispiel ist zu erkennen, dass die grafische Einstellhilfe dem Nutzer zumindest eine zweifache (31, 34), bevorzugt eine dreifache Rückmeldung zur Einstellung (31,32,34) gibt. Die Bedienvorrichtung für ein Beatmungsgerät umfasst eine berührungsempfindliche grafische Anzeige (3, 13, 14, 15) die zumindest zeitweise den Wertebereich für einen Beatmungsparameter (14a ... 14x), hier die Rampensteilheit (30), repräsentiert und zumindest einzelne Werte der Rampensteilheit numerisch darstellt (31). Darüber hinaus sind ein Speicher für den Wert der Rampensteilheit für mindestens einen mit dem Wertebereich verbundenen Datenpunkt und mindestens eine mit dem Datenpunkt schaltlogisch verbundene Stelle (14a ... 14x) der berührungsempfindlichen grafischen Anzeige verwendet. Weiterhin sind eine Schaltlogik , die bei Berührung der, dem Datenpunkt schaltlogisch zugeordneten Stelle der berührungsempfindlichen grafischen Anzeige eine Anzeige von mindestens einem, dem Datenpunkt zugeordneten, Zahlenwert (32) und/oder eines Bestätigungsfeldes (33) für den Zahlenwert veranlasst und eine Schaltlogik, die bei Berührung des Zahlenwertes (32) oder des Betätigungsfeldes (33), diesen Zahlenwert auf den zugeordneten Atemgasparameter anwendet und diesen Zahlenwert mit dem zugeordneten Atemgasparameter in den Speicher schreibt, vorgesehen.

Das Bedienfeld ist in Form eines Zahlenstrahls oder Lineals ausgeführt und der gesamte Wertebereich ist in Form eines Zahlenstrahls oder Balkens auf der Anzeige (13) visualisiert und der visualisierte Zahlenstrahl ist auch als Bedienfeld (14f) ausgebildet. Das Bedienfeld ist über den gesamten visualisierten Einstellbereich berührungsempfindlich und der gewünschte Wert ist mit lediglich einer Berührung des gewünschten Bereiches anwählbar.

Ein Fingerdruck oder eine Berührung innerhalb des Lineals wird bezüglich seiner Position ausgewertet derart, dass man nicht genau eine der Zahlen 1, 2, 3 treffen muss, sondern der Fingerdruck der nächstliegenden Zahl zugeordnet wird. Der erkannte Wert (32) wird in einem Zusatzfeld (14f1) visualisiert. Der erkannte Wert kann zusätzlich mittels der Symbole +/- (14f2,14f3) verstellt werden.

Bevorzugt werden nicht nur einzelne Werte der Rampensteilheit numerisch dargestellt (31), sondern es wird auch der gewählte Wert der Rampensteilheit numerisch dargestellt (33) und es erfolgt auch eine graphische Visualisierung der gewählten Rampensteilheit (34).

Alternativ oder ergänzend kann, wie zu Fig. 7 ausgeführt, auch die Rampe von dem inspiratorischen zu dem exspiratorischen Druck eingestellt werden.

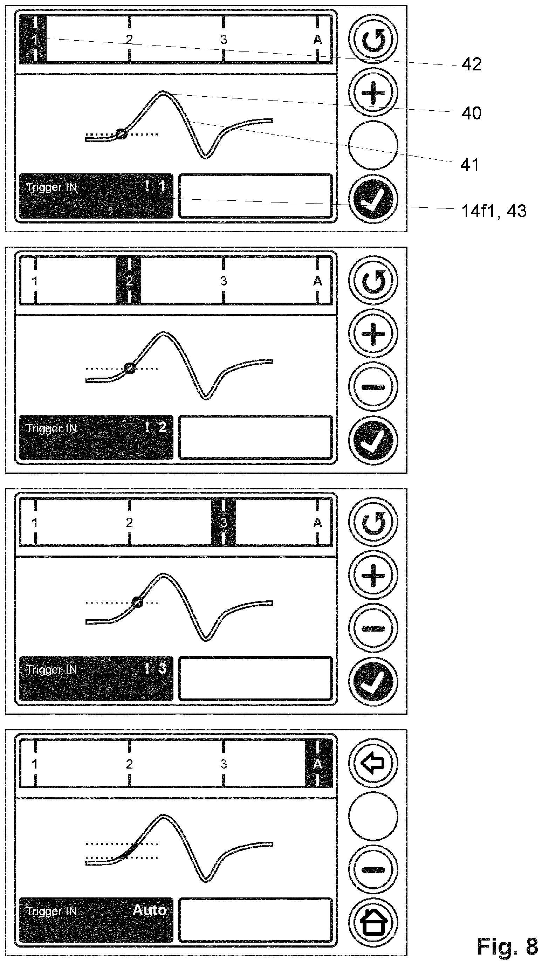

Die Abbildung 8 zeigt, analog zu Fig. 7, die Einstellung der Trigger-Sensitivität (40). Der Schwellwert (41) ist als grüne Linie in der Abbildung schematisch dargestellt. Gegenüber Abbildung 7 sieht man, dass im Slider / Lineal auch Werte ausgewählt werden können, die keinen Zahlenwert darstellen. In diesem Falle "A" für die Stufe Auto. Die Bedienvorrichtung für ein Beatmungsgerät umfasst eine berührungsempfindliche grafische Anzeige (3, 13, 14, 15) die zumindest zeitweise den Wertebereich für einen Beatmungsparameter (14a ... 14x), hier die Trigger-Sensitivität (40), repräsentiert und zumindest einzelne Werte der Trigger-Sensitivität numerisch darstellt (42), sowie einen Speicher für den Wert der Trigger-Sensitivität mindestens einen mit dem Wertebereich verbundenen Datenpunkt. Ebenfalls sind mindestens eine mit dem Datenpunkt schaltlogisch verbundene Stelle (14a ... 14x) der berührungsempfindlichen grafischen Anzeige und eine Schaltlogik, die bei Berührung der, dem Datenpunkt schaltlogisch zugeordneten Stelle der berührungsempfindlichen grafischen Anzeige eine Anzeige von mindestens einem, dem Datenpunkt zugeordneten, Zahlenwert (32) und /oder eines Bestätigungsfeldes (33) für den Zahlenwert veranlasst, verwendet. Ebenfalls vorgesehen ist eine Schaltlogik, die bei Berührung des Zahlenwertes (32) oder des Betätigungsfeldes (33), diesen Zahlenwert auf den zugeordneten Atemgasparameter anwendet und diesen Zahlenwert mit dem zugeordneten Atemgasparameter in den Speicher schreibt, verwendet.

Das Bedienfeld ist in Form eines Zahlenstrahls oder Lineals ausgeführt und der gesamte Wertebereich ist in Form eines Zahlenstrahls oder Balkens auf der Anzeige (13) visualisiert und der visualisierte Zahlenstrahl ist auch als Bedienfeld (14f) ausgebildet. Das Bedienfeld ist über den gesamten visualisierten Einstellbereich berührungsempfindlich und der gewünschte Wert ist mit lediglich einer Berührung des gewünschten Bereiches anwählbar. Ein Fingerdruck oder eine Berührung innerhalb des Lineals wird bezüglich seiner Position ausgewertet derart, dass man nicht genau eine der Zahlen 1, 2, 3 treffen muss, sondern der Fingerdruck der nächstliegenden Zahl zugeordnet wird. Der erkannte Wert (32) wird in einem Zusatzfeld (14f1) visualisiert.

Der erkannte Wert kann zusätzlich mittels der Symbole +/- (14f2,14f3) verstellt werden. Bevorzugt werden nicht nur einzelne Werte der Trigger-Sensitivität numerisch dargestellt (42), sondern es wird auch der gewählte Wert der Trigger-Sensitivität numerisch dargestellt (43) und es erfolgt auch eine graphische Visualisierung (41) der gewählten Trigger-Sensitivität. vorliegend sind drei feste Triggerstufen vorgesehen. Diese können jedoch auch mittels der Symbole +/- (14f2,14f3) verstellt werden, um so den Trigger patientenindividuell feiner abzustimmen. Wird die Stufe "A" für die Stufe Auto gewählt, so verstellt sich der Trigger adaptiv innerhalb vorgegebener Grenzwerte, die auch graphisch visualisiert werden.

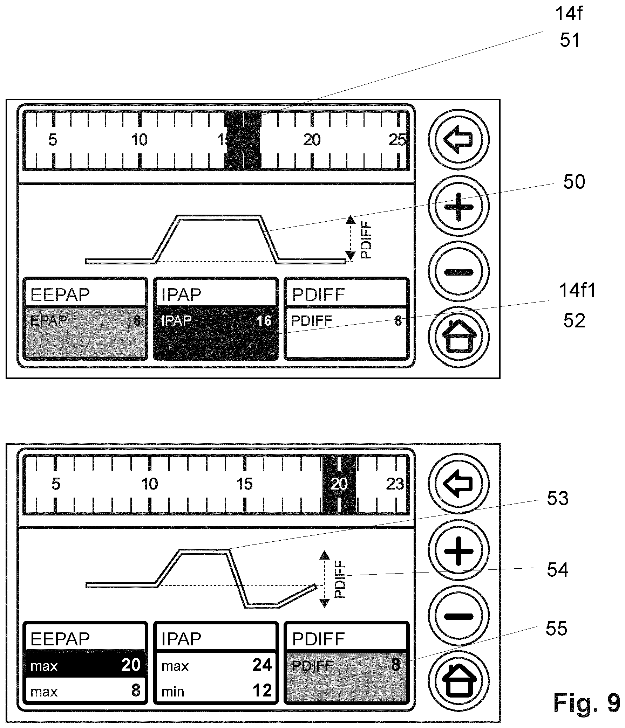

Gemäß Abbildung 9 sieht man die grafische Einstellhilfe für mindestens einen inspiratorischen und einen exspiratorischen Druck. Durch die Position der Druck-Kachel unterhalb der Grafik wird verdeutlicht, welches der inspiratorische und exspiratorische Druck ist, und dass PDIFF den Druckhub (54) darstellt. Die Farbe der Kacheln stellt dar:
- grün: aktuell ausgewählter Parameter, der über Slider oder +/- Tasten verstellt werden kann
- grau: Parameter, der alternativ zur Verstellung ausgewählt werden kann
- schwarz: informativ dargestellter Parameter, der sich als Konsequenz aus den Einstellungen ergibt.

Alternativ könnte man im Slider nicht nur den Wert des aktuell einzustellenden Parameters in einer Farbe darstellen, sondern zusätzlich die Werte weiterer Parameter mit einer Markierung, die sich in Form und/oder Farbe unterscheidet. Die Bedienvorrichtung für ein Beatmungsgerät umfasst eine berührungsempfindliche grafische Anzeige (3, 13, 14, 15) die zumindest zeitweise den Wertebereich für einen Beatmungsparameter (14a ... 14x), hier die Druckwerte (50) IPAP und/oder EPAP und/oder EEPAP, repräsentiert und zumindest einzelne Werte der Trigger-Sensitivität numerisch darstellt (51), einen Speicher für den Druckwert mindestens einen mit dem Wertebereich verbundenen Datenpunkt mindestens eine mit dem Datenpunkt schaltlogisch verbundene Stelle (14a ... 14x) der berührungsempfindlichen grafischen Anzeige eine Schaltlogik , die bei Berührung der, dem Datenpunkt schaltlogisch zugeordneten Stelle der berührungsempfindlichen grafischen Anzeige eine Anzeige von mindestens einem, dem Datenpunkt zugeordneten, Zahlenwert (52) und /oder eines Bestätigungsfeldes für den Zahlenwert veranlasst eine Schaltlogik, die bei Berührung des Zahlenwertes (52) oder des Betätigungsfeldes, diesen Zahlenwert auf den zugeordneten Atemgasparameter anwendet und diesen Zahlenwert mit dem zugeordneten Atemgasparameter in den Speicher schreibt.

Das Bedienfeld ist in Form eines Zahlenstrahls oder Lineals ausgeführt und der gesamte Wertebereich ist in Form eines Zahlenstrahls oder Balkens auf der Anzeige (13) visualisiert und der visualisierte Zahlenstrahl ist auch als Bedienfeld (14f) ausgebildet. Das Bedienfeld ist über den gesamten visualisierten Einstellbereich berührungsempfindlich und der gewünschte Wert ist mit lediglich einer Berührung des gewünschten Bereiches anwählbar. Ein Fingerdruck oder eine Berührung innerhalb des Lineals wird bezüglich seiner Position ausgewertet derart, dass man nicht genau eine der Zahlen treffen muss, sondern der Fingerdruck der nächstliegenden Zahl zugeordnet wird. Der erkannte Wert (52) wird in einem Zusatzfeld (14f1) visualisiert.

Der erkannte Wert kann zusätzlich mittels der Symbole +/- (14f2,14f3) verstellt werden. Bevorzugt werden nicht nur einzelne Druckwerte numerisch dargestellt (52), sondern es wird auch der gewählte Wert numerisch dargestellt und es erfolgt auch eine graphische Visualisierung (53) des gewählten Druckes. Dieser kann jedoch auch mittels der Symbole +/- (14f2,14f3) verstellt werden. Bevorzugt wird zur Information auch der resultierende Druckhub als numerischer Wert (55) dargestellt und graphisch visualisiert (54).

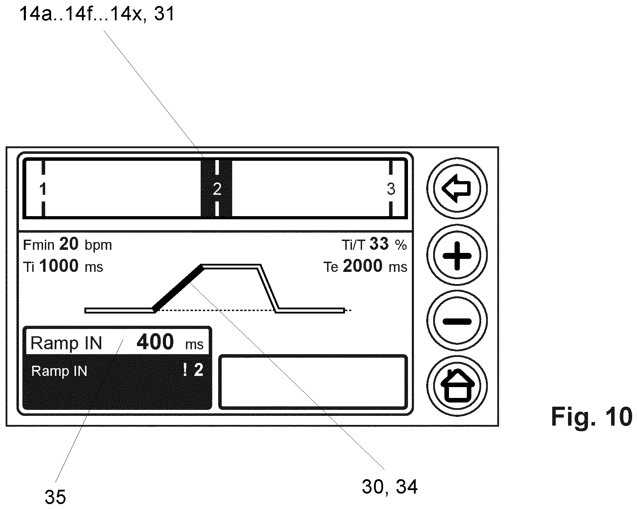

Gemäß Figur 10 wird als Ergänzung zu Figur 7 in Kombination zur aktuellen Rampensteilheit 1, 2 oder 3 (31) noch die Einstellung weiterer damit in logischem Zusammenhang stehender Parameter informativ dargestellt. Dadurch kann der Anwender den aktuell einzustellenden Wert sinnvoll wählen, ohne sich die übrigen Parameter alle merken zu müssen. Als Konsequenz aus aktueller Rampensteilheit und den übrigen Parametern wird die aktuell gültige Rampenzeit in ms (35) berechnet und ebenfalls informativ dargestellt.

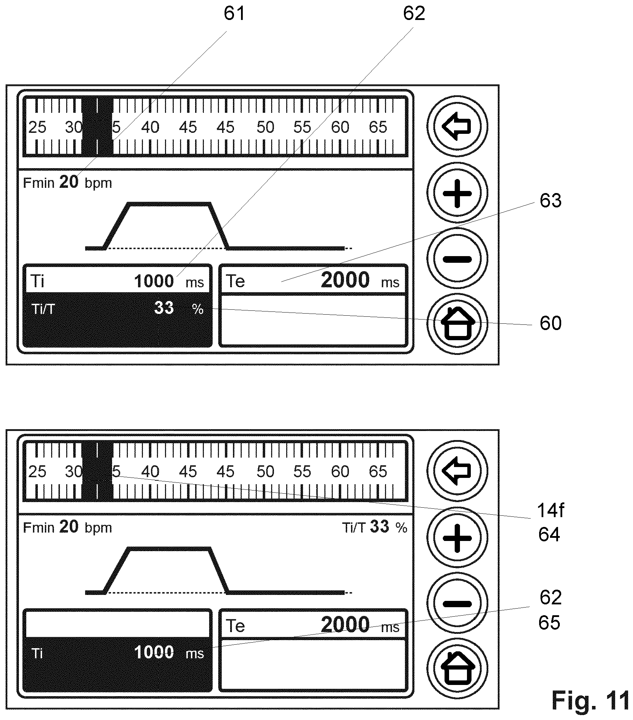

Die Abbildung 11 zeigt die Einstellung des Verhältnisses von inspiratorischer Zeit zur gesamten Atemzugszeit in % (60). Zusätzlich ist die gewählte Atemfrequenz (61) dargestellt. Als Konsequenz aus eingestellter Atemfrequenz und eingestelltem Verhältnis Ti/T werden die Inspirationsdauer Ti (62) und Exspirationsdauer Te (62) berechnet und informativ dargestellt.

In der unteren Hälfte ist eine alternative Ausführung dargestellt, bei der die Inspirationsdauer eingestellt wird, und in Kombination mit der gewählten Atemfrequenz ergeben sich daraus die Exspirationsdauer und Ti/T automatisch. Die Bedienvorrichtung für ein Beatmungsgerät umfasst eine berührungsempfindliche grafische Anzeige (3, 13, 14, 15) die zumindest zeitweise den Wertebereich für einen Beatmungsparameter (14a ... 14x), hier die Inspirationsdauer (62), repräsentiert und zumindest einzelne Werte numerisch darstellt (64), einen Speicher für die Inspirationsdauer mindestens einen mit dem Wertebereich verbundenen Datenpunkt mindestens eine mit dem Datenpunkt schaltlogisch verbundene Stelle (14a ... 14x) der berührungsempfindlichen grafischen Anzeige eine Schaltlogik , die bei Berührung der, dem Datenpunkt schaltlogisch zugeordneten Stelle der berührungsempfindlichen grafischen Anzeige eine Anzeige von mindestens einem, dem Datenpunkt zugeordneten, Zahlenwert (65) und /oder eines Bestätigungsfeldes für den Zahlenwert veranlasst eine Schaltlogik , die bei Berührung des Zahlenwertes (65) oder des Betätigungsfeldes, diesen Zahlenwert auf den zugeordneten Atemgasparameter anwendet und diesen Zahlenwert mit dem zugeordneten Atemgasparameter in den Speicher schreibt.

Das Bedienfeld ist in Form eines Zahlenstrahls oder Lineals ausgeführt und der gesamte Wertebereich ist in Form eines Zahlenstrahls oder Balkens auf der Anzeige (13) visualisiert und der visualisierte Zahlenstrahl ist auch als Bedienfeld (14f) ausgebildet. Das Bedienfeld ist über den gesamten visualisierten Einstellbereich berührungsempfindlich und der gewünschte Wert ist mit lediglich einer Berührung des gewünschten Bereiches anwählbar.

Ein Fingerdruck oder eine Berührung innerhalb des Lineals wird bezüglich seiner Position ausgewertet derart, dass man nicht genau eine der Zahlen treffen muss, sondern der Fingerdruck der nächstliegenden Zahl zugeordnet wird. Der erkannte Wert (52) wird in einem Zusatzfeld (14f1) visualisiert. Der erkannte Wert kann zusätzlich mittels der Symbole +/- (14f2,14f3) verstellt werden. Bevorzugt werden nicht nur einzelne Werte numerisch dargestellt, sondern es wird auch der gewählte Wert numerisch dargestellt und es erfolgt auch eine graphische Visualisierung des gewählten Wertes. Dieser kann jedoch auch mittels der Symbole +/- (14f2,14f3) verstellt werden. Bevorzugt wird zur Information auch die resultierende Ausatemzeit (63) als numerischer Wert (63) dargestellt und / oder graphisch visualisiert.

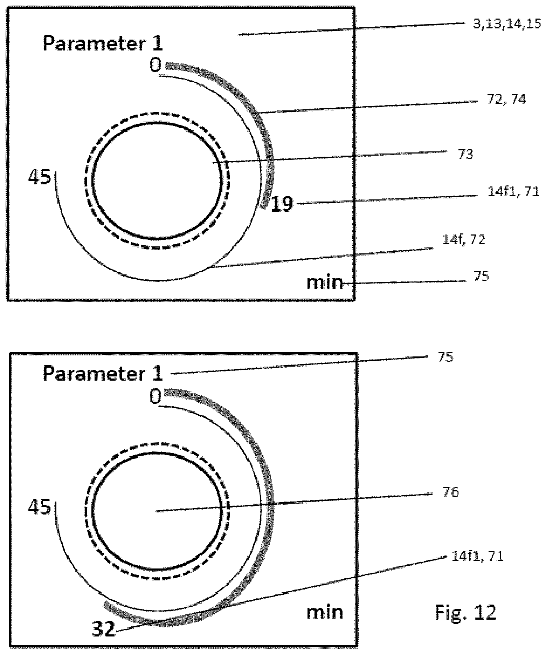

Abbildung 12 zeigt die beschriebene kreisförmige Ausführung des Bedienelementes. Auf einer berührungsempfindlichen Anzeige wird ein Drehrad simuliert, welches mit dem Finger bedient werden kann. Um das Bedienfeld herum ist ein ebenfalls kreisförmiges Anzeigeelement platziert, welches mindestens den aktuell eingestellten Wert darstellt - im Beispiel 19 Minuten -, besonders bevorzugt auch die Wertegrenzen bzw. den Wertebereich - im Beispiel 0 bis 45 Minuten. Die Darstellung erfolgt als Zahl und besonders bevorzugt zusätzlich durch eine farbige und/oder dickere Markierung (74), die die aktuellen Werte in Relation zum gesamten Wertebereich darstellt.

Die Bedienvorrichtung für ein Beatmungsgerät umfasst eine berührungsempfindliche grafische Anzeige (3, 13, 14, 15) die zumindest zeitweise den Wertebereich für einen Beatmungsparameter (14a ... 14x) repräsentiert und zumindest einzelne Werte numerisch darstellt (71), einen Speicher für mindestens einen mit dem Wertebereich verbundenen Datenpunkt mindestens eine mit dem Datenpunkt schaltlogisch verbundene Stelle (14a ... 14x) der berührungsempfindlichen grafischen Anzeige eine Schaltlogik , die bei Berührung der, dem Datenpunkt schaltlogisch zugeordneten Stelle der berührungsempfindlichen grafischen Anzeige eine Anzeige von mindestens einem, dem Datenpunkt zugeordneten, Zahlenwert (71) und /oder eines Bestätigungsfeldes für den Zahlenwert veranlasst eine Schaltlogik, die bei Berührung des Zahlenwertes (71) oder des Betätigungsfeldes, diesen Zahlenwert auf den zugeordneten Atemgasparameter anwendet und diesen Zahlenwert mit dem zugeordneten Atemgasparameter in den Speicher schreibt.

Das Bedienfeld ist in Form eines Drehknopfes oder Drehrades (73) ausgeführt und der gesamte oder ein teilweiser Wertebereich ist in Form eines Zahlenkranzes (72) auf der Anzeige (13) visualisiert und der visualisierte Zahlenkranz ist auch als Bedienfeld (14f) ausgebildet. Das Bedienfeld ist über den gesamten visualisierten Einstellbereich berührungsempfindlich und der gewünschte Wert ist mit lediglich einer Berührung des gewünschten Bereiches anwählbar. Ein Fingerdruck oder eine Berührung innerhalb des Zahlenkranzes wird bezüglich seiner Position ausgewertet derart, dass man nicht genau eine der Zahlen treffen muss, sondern der Fingerdruck der nächstliegenden Zahl zugeordnet wird.

Alternativ wird ein streichen über den Zahlenkranz als Einstellvorgang erkannt und ein stoppen der Streichbewegung als Auswahl. Derjenige Wert der beim Stoppen der Bewegung erkannt wird - der erkannte Wert (71) - wird in einem Zusatzfeld (14f1) visualisiert. Der erkannte Wert kann zusätzlich mittels der Symbole +/- (14f2,14f3) verstellt werden. Bevorzugt werden nicht nur einzelne Werte numerisch dargestellt, sondern es wird auch der gewählte Wert numerisch dargestellt und es erfolgt auch eine graphische Visualisierung des gewählten Wertes. Dieser kann jedoch auch mittels der Symbole +/- (14f2,14f3) verstellt werden.

Besonders bevorzugt wird der aktuell einzustellende Parameter (75) mit Name und/oder einem international verständlichen Symbol (75) und/oder seiner Einheit (75) dargestellt. Im dargestellten Beispiel könnte der Parameter die Rampenzeit als Einschlafhilfe eines Therapiegerätes sein, die in Minuten dargestellt wird. Alternativ dazu können u.a. Therapiedrücke oder die Leistungsstufen eines Atemluftbefeuchters dargestellt und verstellt werden.

Führt der Anwender mit dem Finger eine Drehbewegung auf dem dargestellten Drehrad aus, vorzugsweise im Uhrzeigersinn, so vergrößert sich der gewählte Wert, im Beispiel von 19 auf 32 Minuten. Eine Drehbewegung in die Gegenrichtung führt zu einer Verringerung des gewählten Wertes. Ist der beabsichtigte Wert erreicht, kann er übernommen und vom Gerät angewendet werden. Dies erfolgt typischerweise entweder nach Ablauf einer Wartezeit ohne weitere Verstellung oder nach Drücken einer Bestätigungstaste / Bestätigungsfläche (76), die z. B. mit "Übernehmen", "Ok", "Anwenden", einem Hakensymbol oder ähnlichem gekennzeichnet ist. Diese befindet sich besonders bevorzugt in der Mitte des dargestellten Drehrades.

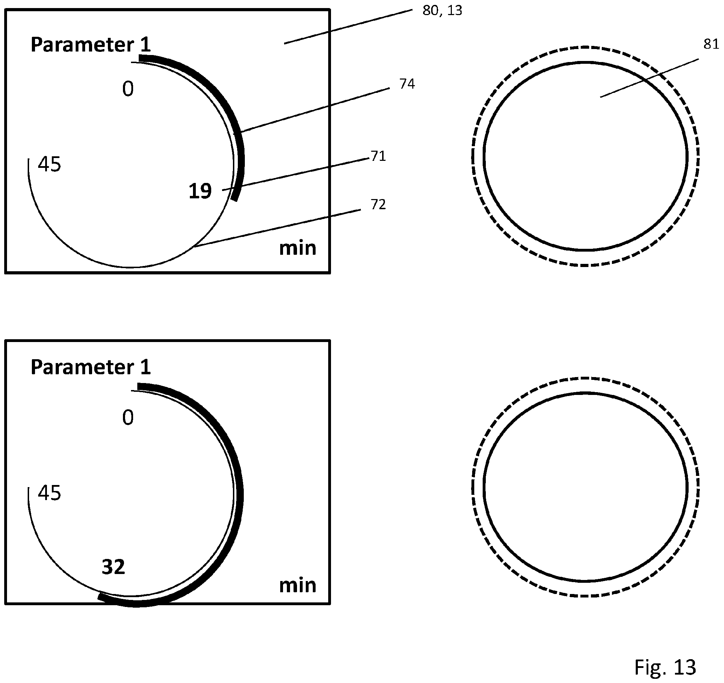

Abbildung 13 zeigt eine alternative Ausführungsform. Hier befinden sich das Bedienelement "Drehrad" und das kreisförmige Anzeigeelement nebeneinander oder untereinander. Dies ist ein bevorzugte Ausführungsform, falls kein berührungsempfindliches Anzeigeelement verwendet wird, sondern nur ein grafisches Anzeigeelement (80) mit einem separat ausgeführten mechanischen Dreh-Drück-Knopf (81). Diese Ausführung bietet einen Vorteil, da der mechanische Dreh-Drück-Knopf (81) wegen der Haptik oder besseren Bedienbarkeit bevorzugt für feine Einstellungen verwendet werden kann. Die vom mechanischen Dreh-Drück-Knopf (81) entkoppelte graphische Visualisierung des Einstellvorganges und/oder der ausgewählten Wertes und/oder des zur Verfügung stehenden Wertebereiches bietet den Vorteil, dass eine größere und verbesserte Darstellung gewählt werden kann, als es eine Skala neben dem mechanischen Dreh-Drück-Knopf (81) bieten könnte.

Das Bestätigen eines gewählten Wertes geschieht hier besonders bevorzugt durch Drücken des Dreh-Drück-Knopfes (81).

Ansonsten ist die Art der Darstellung und Einstellung mit dem Ausführungsbeispiel der Fig. 12 vergleichbar, weshalb die Beschreibung zur Fig. 12 auch für das Beispiel der Fig. 13 herangezogen werden kann.

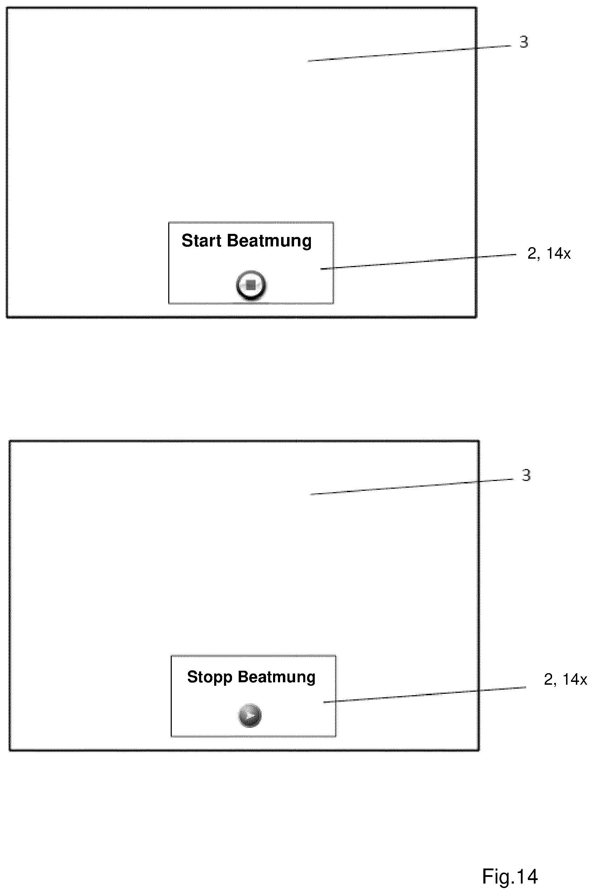

Erfindungsgemäß kann eine (Start/Stopp) Bedienfläche (14x) auf dem Touchscreen oder ein mechanisches (Start/Stopp) Bedienelement (2) gemäß Figur 14 vorgesehen sein, dessen Betätigung das Bedien- und Informationssystem (3) veranlasst über die Steuereinheit die Beatmung zu starten oder zu beenden.

Erfindungsgemäß ist vorgesehen, dass die (Start/Stopp) Bedienfläche (14x) auf dem Touchscreen situationsabhängig unterschiedlich gestaltet ist; beispielsweise wenn die Beatmung nicht aktiv ist, aber gestartet werden kann, so ist die (Start/Stopp) Bedienfläche (14x) auf dem Touchscreen beispielsweise zumindest teilweise grün eingefärbt oder weist ein Startsymbol auf und weist beispielsweise ergänzend einen schriftliche Information "Beatmung Start" auf. Wenn die Beatmung aktiv ist und gestoppt werden kann, so ist die (Start/Stopp) Bedienfläche (14x) auf dem Touchscreen zumindest teilweise rot eingefärbt oder weist ein Stoppsymbol auf und weist beispielsweise ergänzend einen schriftliche Information "Beatmung Stopp" auf.

Dabei ist beispielsweise vorgesehen, dass die (Start/Stopp) Bedienfläche (14x) ansonsten unverändert an immer derselben Position auf dem Touchscreen und/oder in immer derselben Größe erscheint.

Bei Bestätigung der (Start/Stopp) Bedienfläche (14x) auf dem Touchscreen zum Stoppen der Beatmung "Beatmung Stopp", werden von der Steuereinheit aktuelle Einstellungen für die Beatmung, wie beispielsweise aktuelle Druckwerte, abrufbar gespeichert und auf Wieder-Betätigung der (Start/Stopp) Bedienfläche (14x) hin - zum Starten der Beatmung - wieder ausgelesen und für die Beatmung aktiviert, insbesondere sofern während des Beatmungs-Stopps keine Veränderungen an den Einstellungen für die Beatmung vorgenommen wurden.

## Patentansprüche

1. Bedien- und Informationssystem für ein Beatmungsgerät (1) mit einer Anzeige (13) zur Darstellung von Informationen und zur Darstellung von Bedienfeldern (14a-x) für den Anwender und zumindest einem berührungsempfindlichen Eingabefeld (14a1 - 14x1) in räumlicher Nähe zum dargestellten Bedienfeld (14a-x) wobei im Bereich der Anzeige ein erstes Bedienfeld (14a) und ein zweites Bedienfeld (14b) dargestellt werden, eine Verarbeitungseinheit, welche mit der Anzeige und der berührungsempfindlichen Eingabeeinheit gekoppelt ist, wobei die Verarbeitungseinheit ausgestaltet ist, eine Bedienung des ersten Bedienfeldes (14a) über die berührungsempfindliche Eingabeeinheit zu erfassen und in Abhängigkeit davon die Steuereinheit zu veranlassen die dem Bedienfeld zugeordnete Funktion auszuführen oder darzustellen, wobei eine Bedienung des zweiten Bedienfeldes (14b) über die berührungsempfindliche Eingabeeinheit von der Verarbeitungseinheit erfasst wird und die Steuereinheit daraufhin veranlasst wird ein kontextabhängiges Untermenü aufzurufen, wobei das Untermenü eine Auswahl von betätigbaren Einstellfunktionen für das Menü umfasst, wobei die Steuereinheit eingestellte Parameter im Bereich des Menüs visualisiert und über Steuerbefehle an einen zugeordneten Aktor zur Anwendung bringt und wobei der Steuereinheit ein Speicher zugeordnet ist und eingestellte Parameter oder Werte von der Steuereinheit in den Speicher geschrieben werden, wobei der Speicher zumindest die zuletzt eingegebenen und/oder angewendeten Werte speichert **dadurch gekennzeichnet, dass** die Steuereinheit Strom- oder Widerstands- oder Spannungsänderungen im Bereich von Schnittstellen oder von Anschlüssen zum Anfeuchter registriert und so erkennt, wenn ein Anfeuchter an den Anfeuchteranschluss adaptiert wird und daraufhin von der Steuereinheit über die Anzeige (13) ein neues Menü mit Bedienfeld für den Anfeuchter (14f) dargestellt wird, wobei das Menü (14f) für die Anfeuchtersteuerung im Wesentlichen an einer bisher nicht mit einem Menü belegten Stelle der Anzeige dargestellt wird, wobei benachbart zum Bedienfeld (14f) zusätzliche Bedienfelder (14f1) visualisiert werden, die zunächst nicht aktiv sind, wobei eine Betätigung des Bedienfeldes (14f) von der Verarbeitungseinheit erfasst wird, welche die Steuereinheit veranlasst die Bedienfelder (14f1) zu aktivieren und wobei über diese eine Einstellung einer Anfeuchterheizstufe erfolgt und eine gewählte Anfeuchterstufe daraufhin im Bedienfeld (14f) angezeigt wird und zudem der gewählte Wert von der Steuereinheit über ein Steuersignal an den Anfeuchter eingestellt und angewandt wird.

2. Bedien- und Informationssystem nach zumindest einem der vorhergehenden Ansprüche **dadurch gekennzeichnet, dass** die Steuereinheit über die Schnittstelle (8) angeschlossene Speichermedien erkennt und ein angeschlossenes Speichermedium im Bereich der berührungsempfindlichen Eingabeeinheit als Bedienfeld darstellt und so auch therapiefremde Daten in das Beatmungsgerät geladen werden und/oder von diesem ausgeführt werden.

3. Bedien- und Informationssystem nach zumindest einem der vorhergehenden Ansprüche **dadurch gekennzeichnet, dass** die Steuereinheit Strom- oder Widerstands- oder Spannungsänderungen im Bereich eines Heizelementes des Anfeuchters registriert und so einen sinkenden oder geringen Wasserstand anhand eines erhöhten Strom- oder Widerstands- oder Spannungswertes eines Heizelementes erkennt und dies die Steuereinheit veranlasst, ein Symbol oder eine Textmeldung die einen niedrigen Wasserstand symbolisieren oder benennen im Bereich der Anzeige einzublenden.

4. Bedien- und Informationssystem nach zumindest einem der vorhergehenden Ansprüche, umfassend:
eine grafische Anzeige (3, 13, 14, 15) die zumindest zeitweise den Wertebereich für einen Beatmungsparameter (14a ... 14x) repräsentiert und zumindest einzelne Werte numerisch darstellt, einen Speicher (21) für Beatmungsparameter-Werte mindestens einen mit dem Wertebereich verbundenen Datenpunkt mindestens eine mit dem Datenpunkt schaltlogisch verbundene Stelle (14a ... 14x) der grafischen Anzeige
eine Schaltlogik, die bei Betätigung der, dem Datenpunkt schaltlogisch zugeordneten Stelle des mechanischen Bedienelementes eine Anzeige von mindestens einem, dem Datenpunkt zugeordneten, Zahlenwert und /oder eines Bestätigungsfeldes für den Zahlenwert veranlasst
eine Schaltlogik , die bei Betätigung der, dem Datenpunkt schaltlogisch zugeordneten Stelle des mechanischen Bedienelementes diesen Zahlenwert auf den zugeordneten Atemgasparameter anwendet und diesen Zahlenwert mit dem zugeordneten Atemgasparameter in den Speicher (21) schreibt wobei kein berührungsempfindliches Anzeigeelement verwendet wird, sondern nur ein grafisches Anzeigeelement (80) mit einem separat ausgeführten mechanischen Bedienelement (81) und die mittels des mechanischen Bedienelementes vorgenommen Einstellungen auf dem grafischen Anzeigeelement (80) graphisch visualisiert werden und wobei auf dem grafischen Anzeigeelement (80) die Veränderung der Werte während des Einstellvorganges über das mechanische Bedienelement (81) und/oder der ausgewählte Werte und/oder der zur Verfügung stehenden Wertebereiches visualisiert werden.

5. Bedien- und Informationssystem nach zumindest einem der vorhergehenden Ansprüche **dadurch gekennzeichnet, dass** ein Arzt und ein Patienten-Menü vorgesehen sind wobei das Arzt-Menü alle wichtigen Einstellmöglichkeiten bietet während das Patientenmenü lediglich reduzierte Einstellmöglichkeiten bietet und eine Freigabefunktion für den Arzt vorgesehen ist, wobei dem Arzt erst nach Authentifizierung alle Einstellmöglichkeiten freigeschaltet werden.

6. Bedien- und Informationssystem nach zumindest einem der vorhergehenden Ansprüche **dadurch gekennzeichnet, dass** das Bedienfeld in Form eines Zahlenstrahls oder Lineals ausgeführt ist und der gesamte Wertebereich in Form eines Zahlenstrahls oder Balkens auf der Anzeige (13) visualisiert ist und der visualisierte Zahlenstrahl auch als Bedienfeld (14f) ausgebildet ist wobei das Bedienfeld über den gesamten visualisierten Einstellbereich berührungsempfindlich ist und der gewünschte Wert mit lediglich einer Berührung des gewünschten Bereiches anwählbar ist.

7. Bedien- und Informationssystem nach Anspruch 6 **dadurch gekennzeichnet, dass** ein Fingerdruck oder eine Berührung innerhalb des Lineals bezüglich seiner Position ausgewertet wird derart, dass der Fingerdruck der nächstliegenden Zahl zugeordnet wird und der erkannte Wert (52) in einem Zusatzfeld (14f1) visualisiert wir, wobei der erkannte Wert zusätzlich mittels der Symbole +/- (14f2,14f3) verstellt werden kann.

## Claims

1. An operating and information system for a breathing apparatus (1), having a display (13) for displaying information and for displaying operating fields (14a-x) for the user and at least one touch-sensitive input field (14a1 - 14x1) in spatial proximity to the displayed operating field (14a-x), wherein a first operating field (14a) and a second operating field (14b) are displayed in the region of the display, a processing unit which is coupled to the display and to the touch-sensitive input unit, wherein the processing unit is configured to detect an operation of the first operating field (14a) via the touch-sensitive input unit and, on the basis thereof, to cause the control unit to carry out or display the function associated with the operating field, wherein an operation of the second operating field (14b) is detected by the processing unit via the touch-sensitive input unit and the control unit is then caused to call up a context-dependent submenu, the submenu comprising a selection of actuatable adjustment functions for the menu, the control unit visualizing adjusted parameters in the region of the menu and applying them via control commands to an associated actuator and wherein a memory is associated with the control unit and adjusted parameters or values are written to the memory by the control unit, the memory storing at least the values input and/or applied last, **characterized in that** the control unit registers current or resistance or voltage changes in the region of interfaces or connections to the humidifier and thus detects when a humidifier is adapted to the humidifier connection and a new menu having an operating field for the humidifier (14f) is then displayed by the control unit via the display (13), wherein the menu (14f) for controlling the humidifier is displayed substantially at a position on the display which was not previously occupied by a menu, wherein additional operating fields (14f1) which are initially not active are visualized adjacent to the operating field (14f), actuation of the operating field (14f) being detected by the processing unit which causes the control unit to activate the operating fields (14f1) and the latter being used to adjust a humidifier heating level and a selected humidifier level then being displayed in the operating field (14f), and the selected value being additionally adjusted and used by the control unit via a control signal to the humidifier.

2. The operating and information system according to at least one of the preceding claims, **characterized in that** the control unit detects storage media connected via the interface (8) and displays a connected storage medium as an operating field in the region of the touch-sensitive input unit and data which are extraneous to therapy are thus also loaded into the breathing apparatus and/or executed by the latter.

3. The operating and information system according to at least one of the preceding claims, **characterized in that** the control unit registers current or resistance or voltage changes in the region of a heating element of the humidifier and thus detects a falling or low water level on the basis of an increased current or resistance or voltage value of a heating element and this causes the control unit to insert a symbol or a text message symbolizing or indicating a low water level in the region of the display.

4. The operating and information system according to at least one of the preceding claims, comprising:
a graphical display (3, 13, 14, 15) which at least occasionally represents the range of values for a breathing parameter (14a ... 14x) and numerically displays at least individual values, a memory (21) for breathing parameter values,
at least one data point associated with the range of values,
at least one position (14a ... 14x) on the graphical display which is associated with the data point using switching logic,
a switching logic which, when the position on the mechanical operating element which is associated with the data point using switching logic is touched, causes at least one numerical value associated with the data point and/or a confirmation field for the numerical value to be displayed,
a switching logic which, when the position on the mechanical operating element which is associated with the data point using switching logic is touched, applies this numerical value to the associated respiratory gas parameter and writes this numerical value, with the associated respiratory gas parameter, to the memory (21), wherein no touch-sensitive display element is used, but only a graphical display element (80) having a separate mechanical operating element (81) and the adjustments made using the mechanical operating element are graphically visualized on the graphical display element (80) and wherein the change in values during the adjustment process using the mechanical operating element (81) and/or the selected values and/or the available range of values is/are visualized on the graphical display element (80).

5. The operating and information system according to at least one of the preceding claims, **characterized in that** a doctor and a patient menu are provided, wherein the doctor menu offers all the important adjustment possibilities, whilst the patient menu merely offers reduced adjustment possibilities and a clearance function is provided for the doctor, wherein all of the adjustment possibilities are not enabled for the doctor until after authentication.

6. The operating and information system according to at least one of the preceding claims, **characterized in that** the operating field is executed in the form of a number line or ruler and the entire range of values is visualized on the display (13) in the form of a number line or bar and the visualized number line is also configured as an operating field (14f), wherein the operating field is touch-sensitive over the entire visualized adjustment range and the desired value can be selected by merely touching the desired range.

7. The operating and information system according to Claim 6, **characterized in that** finger pressure or touching inside the ruler is evaluated with respect to its position in such a manner that the finger pressure is associated with the closest number and the detected value (52) is visualized in an additional field (14f1), wherein the detected value can additionally be adjusted using the symbols +/- (14f2,14f3).

## Revendications

1. Système de commande et d'information pour un appareil respiratoire (1) avec un écran (13) pour l'affichage d'informations et pour l'affichage de champs de commande (14a-x) destinés à l'utilisateur et au moins un champ de saisie tactile (14a1 - 14x1) à proximité du champ de commande (14a-x) affiché, la région de l'écran affichant un premier champ de commande (14a) et un deuxième champ de commande (14b), avec une unité de traitement accouplée à l'écran et à l'unité de saisie tactile, dans lequel l'unité de traitement est conçue pour détecter une commande du premier champ de commande (14a) par le biais de l'unité de saisie tactile et pour amener l'unité de contrôle à exécuter ou afficher la fonction associée au champ de commande en fonction de celle-ci, dans lequel une commande du deuxième champ de commande (14b) par le biais de l'unité de saisie tactile est détectée par l'unité de traitement et l'unité de contrôle est ainsi amenée à accéder à un sous-menu contextuel, dans lequel le sous-menu comporte un choix de fonctions de réglage activables pour le menu, dans lequel l'unité de contrôle visualise des paramètres réglés dans la région du menu et met ceux-ci en application par des ordres de contrôle transmis à un acteur associé et dans lequel une mémoire est associée à l'unité de contrôle et des paramètres ou valeurs réglés sont écrits dans la mémoire par l'unité de contrôle, dans lequel la mémoire enregistre au moins les valeurs entrées et/ou appliquées en dernier, **caractérisé en ce que** l'unité de contrôle relève des variations de courant ou de résistance ou de tension dans la région d'interfaces ou de raccords à l'humidificateur et reconnaît ainsi lorsqu'un humidificateur est adapté au raccord d'humidificateur et un nouveau menu avec un champ de commande pour l'humidificateur (14f) est alors affiché sur l'écran (13) par l'unité de contrôle, dans lequel le menu (14f) pour le contrôle de l'humidificateur est affiché essentiellement dans une zone de l'écran jusque-là non occupée par un menu, dans lequel des champs de commande supplémentaires (14f1) initialement inactifs sont visualisés à proximité du champ de commande (14f), dans lequel une activation du champ de commande (14f) est détéctée par l'unité de traitement, laquelle amène l'unité de contrôle à activer les champs de commande (14f1), et dans lequel un niveau de chauffage d'humidificateur est réglé par le biais de celle-ci et un niveau d'humidificateur sélectionné est alors affiché dans le champ de commande (14f) et la valeur sélectionnée est également réglée et appliquée par l'unité de contrôle par le biais d'un signal de contrôle transmis à l'humidificateur.

2. Système de commande et d'information selon l'une au moins des revendications précédentes, **caractérisé en ce que** l'unité de contrôle identifie des supports d'enregistrement raccordés par le biais de l'interface (8) et affiche un support d'enregistrement raccordé, sous la forme d'un champ de commande dans la région de l'unité de saisie tactile, des données non thérapeutiques étant ainsi chargées dans l'appareil respiratoire et/ou exécutées par celui-ci.

3. Système de commande et d'information selon l'une au moins des revendications précédentes, **caractérisé en ce que** l'unité de contrôle relève des variations de courant ou de résistance ou de tension dans la région d'un élément de chauffage de l'humidificateur et reconnaît ainsi un niveau d'eau en baisse ou faible à l'aide d'une valeur de courant ou de résistance ou de tension élevée d'un élément de chauffage, amenant ainsi l'unité de contrôle à représenter un symbole ou un message texte symbolisant ou mentionnant un niveau d'eau bas dans la région de l'écran.

4. Système de commande et d'information selon l'une au moins des revendications précédentes, comportant :
un écran graphique (3, 13, 14, 15) représentant au moins temporairement la plage de valeurs pour un paramètre d'assistance respiratoire (14a... 14x) et affichant numériquement au moins des valeurs individuelles, une mémoire (21) pour des valeurs de paramètres d'assistance respiratoire,
au moins un point de données relié à la plage de valeurs,
au moins une zone (14a... 14x) de l'écran graphique reliée par circuiterie au point de données,
une circuiterie permettant d'afficher au moins une valeur numérique associée au point de données et/ou un champ de commande pour la valeur numérique lors de l'activation de la zone de l'élément de commande mécanique associée par circuiterie au point de données,
une circuiterie appliquant cette valeur numérique au paramètre de gaz respiratoire associé lors de l'activation de la zone de l'élément de commande mécanique associée par circuiterie au point de données et écrivant cette valeur numérique avec le paramètre de gaz respiratoire associé dans la mémoire (21), dans lequel aucun élément d'écran tactile n'est utilisé, mais seulement un élément d'écran graphique (80) avec un élément de commande mécanique (81) réalisé séparément, et les réglages effectués au moyen de l'élément de commande mécanique sur l'élément d'écran graphique (80) sont visualisés graphiquement, et dans lequel la modification des valeurs pendant le processus de réglage par le biais de l'élément de commande mécanique (81) et/ou des valeurs sélectionnées et/ou de la plage de valeurs disponible est visualisée sur l'élément d'écran graphique (80).

5. Système de commande et d'information selon l'une au moins des revendications précédentes, **caractérisé en ce qu'**il est prévu un menu de médecin et de patient, dans lequel le menu de médecin propose toutes les possibilités de réglages importantes, tandis que le menu de patient ne propose que des possibilités de réglage réduites, et dans lequel il est prévu une fonction d'autorisation pour le médecin, l'ensemble des possibilités de réglage n'étant débloqué pour le médecin qu'après authentification.

6. Système de commande et d'information selon l'une au moins des revendications précédentes, **caractérisé en ce que** le champ de commande est réalisé sous la forme d'un axe gradué ou d'une règle et l'ensemble de la plage de valeurs est visualisé sous la forme d'un axe gradué ou d'une barre sur l'écran (13) et l'axe gradué visualisé est également conçu comme un champ de commande (14f), le champ de commande étant tactile sur l'ensemble de la région de réglage visualisée et la valeur souhaitée pouvant être sélectionnée par simple effleurement de la région souhaitée.

7. Système de commande et d'information selon la revendication 6, **caractérisé en ce qu'**une pression du doigt ou un effleurement à l'intérieur de la règle est évalué quant à sa position, de telle façon que la pression du doigt est associée au nombre le plus proche et la valeur reconnue (52) est visualisée dans un champ complémentaire (14f1), la valeur reconnue pouvant en outre être modifiée au moyen des symboles +/(14f2, 14f3).
